# EUROPEAN PATENT APPLICATION

(11) **EP 1 510 184 A2**
(43) Date of publication of application: **02.03.2005**
(21) Application number: 04020502.3
(22) Date of filing: 27.08.2004
(51) Int. Cl.: A61C 5/00, A61C 13/00

(54) **Dental object to be bonded by a resin-based cement and method of producing the same**

(30) Priority: 29.08.2003 JP 2003305853
(71) Applicant: Matsumoto Dental University, Nagano 399-0781 (JP)
(72) Inventor: Ito, Michio, Shiojiri Nagano 399-0781 (JP)
(74) Representative: VOSSIUS & PARTNER

(57) **Abstract**

A cementing object (11) comprises a cementing component (12) having a cementing surface (13), powder particles (23) fixedly attached to the cementing surface, and a resin coat (25) coating a surface of each particle on the cementing surface. By sandblasting, the powder particles are fixedly attached to the cementing component with the powder particles partially embedded into the cementing surface. The cementing object is bonded to a mating cementing object (31) via a resin-based cement (41). The resin and the resin-based cement form a chemical bond to achieve a strong bonding strength between the cementing object and the mating cementing object.

## Description

This application claims priority to prior Japanese patent application JP 2003-305853, the disclosure of which is incorporated herein by reference.

This invention relates to a cementing object to be cemented to a mating cementing object via a resin-based cement and to a method of producing the cementing object.

In the dental field, it is a general practice to bond or cement a cementing component to a mating cementing object through a cement. For example, the cementing component may be an orthodontic bracket, a crown or a bridge as a prosthetic restoration, or an inlay for restoration, which is made of a metal or an alloy. The mating cementing object may be a tooth.

In order to bond the cementing component and the mating cementing object, the cementing component is at first subjected to sandblasting by spraying powder particles onto a cementing surface of the cementing component. Thereafter, the cementing surface of the cementing component is bonded to the mating cementing object via the cement.

However, since the cementing component and the cement are different substances so that a high bonding strength can not be achieved.

In the dental field, use is made of a technique of building up a support post for fitting an implant crown. In this technique, a metal wire to serve as a core rod is subjected to sandblasting. Then, a metal-bonding primer is applied on the metal wire. Further, a resin is applied on the primer. Thus, the core rod is formed. The support post is produced by molding a composite resin around the core rod (for example, see JP-A-2000-237209).

However, in the above-mentioned technique of building up the support post, the bonding strength between the implant crown and the support post is insufficient because the support post is obtained by merely applying the metal-bonding primer and the resin after sandblasting the metal wire.

It is therefore an object of this invention to provide a cementing object improved in bonding strength between the cementing object and a mating cementing object.

According to this invention, there is provided a cementing object to be bonded to a mating cementing object via a resin-based cement, the cementing object comprising a cementing component which is formed by a metal or an alloy and which has a cementing surface; powder particles fixedly attached to the cementing component with the powder particles partially embedded into the cementing surface of the cementing component; and a resin coat coating a surface of each particle except a part embedded into the cementing surface.

The powder particles are one of silicon carbide, glass beads, and alumina.

The cementing object is a dental object and the cementing component is selected from a group including a crown or a bridge as a prosthetic restoration, an inlay for restoration, an implant super structure, a cast plate, and an orthodontic bracket each of which is made of a metal or an alloy.

According to this invention, where is also provided a method of producing a cementing object to be cemented to a mating cementing object via a resin-based cement, the method comprising the steps of preparing a cementing component which is formed by a metal or an alloy and which has a cementing surface; preparing resin-coated powder particles by mixing powder particles with resin; and spraying the resin-coated powder particles onto the cementing surface of the cementing component to fixedly attach the resin-coated powder particles to the cementing component with the particles partially embedded into the cementing surface.

According to this invention, there is also provided a cementing powder for use in a dental cementing object, comprising powder particles of silicon carbide, glass beads, or alumina, and resin coats coating the powder particles.

Now, description will be made of this invention with reference to the drawing.
Fig. 1 is a sectional view showing a cementing object according to an embodiment of this invention;
Fig. 2 is an enlarged sectional view of the cementing object illustrated in Fig. 1;
Fig. 3 is a sectional view of the cementing object in Fig. 1 when it is bonded to a mating cementing object;
Fig. 4 is a sectional view of resin-coated powder particles to be sprayed onto a cementing surface of a cementing component illustrated in Fig. 1;
Fig. 5 is a graph showing a bonding strength of a cementing object of a comparative sample 1;
Fig. 6 is a graph showing a bonding strength of a cementing object of a comparative sample 2;
Fig. 7 is a graph showing a bonding strength of a cementing object of a comparative sample 3; and
Fig. 8 is a graph showing a bonding strength of the cementing object according to this invention as compared with the cementing objects of the comparative samples 1 to 3 and a comparative sample 4.

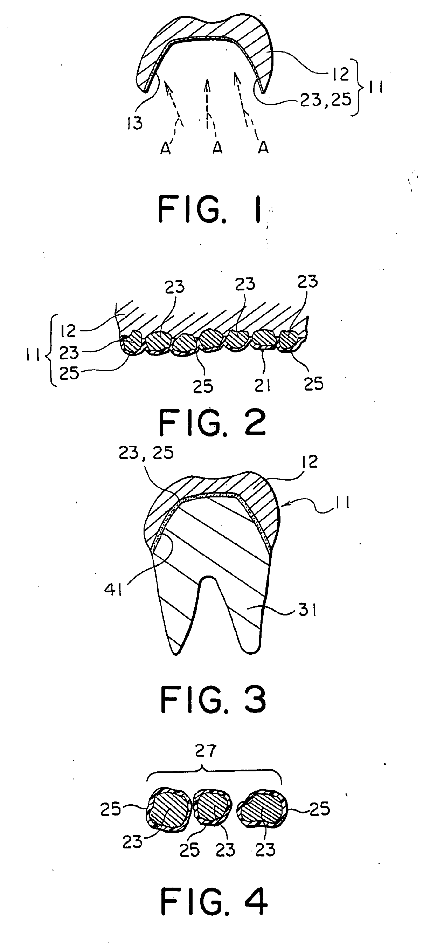

Referring to Figs. 1 and 2, a cementing object according to an embodiment of this invention comprises a cementing component 12 having a cementing surface 13, powder particles 23 fixedly attached to the cementing surface 13, and a resin coat 25 coating a surface of each particle 23 on the cementing surface 13.

The cementing component 12 in this embodiment is a crown made of an alloy. The powder particles 23 are prepared from alumina (Al₂O₃).

As shown in Fig. 2, the powder particles 23 are fixedly attached to the cementing component 12 with the powder particles 23 partially embedded into the cementing surface 13.

Referring to Fig. 3, the cementing object 11 is bonded to a mating cementing object 31 via a resin-based cement 41. The resin coat 25 and the resin-based cement 41 form a chemical bond. In this embodiment, the mating cementing object 31 is a tooth.

Referring to Fig. 4, a method of producing the cementing object 11 will be described. As illustrated in Fig. 4, resin-coated powder 27 to be sprayed onto the cementing surface 13 of the cementing component 12 comprises the powder particles 23 coated with the resin coat 25.

At first, powder as an aggregate of the powder particles 23 and resin powder are mixed. During mixing, heat treatment is carried out so that the surface of each particle 23 of the powder is coated with the resin coat 25 to obtain the resin-coated powder 27.

As depicted by arrows A in Fig. 1, the resin-coated powder 27 is sprayed onto the cementing surface 13 of the cementing component 12 by the use of a compressed gas, such as compressed air. The particles 23 of the resin-coated powder 27 thus sprayed are fixedly attached to the cementing component 12 as illustrated in Fig. 2.

When the resin-coated powder 27 is sprayed onto the cementing surface 13, each particle 23 is press-fitted and partially embedded into the cementing component 12. At this time, a part of the resin coat 25 coating the surface of the particle 23 is shattered into pieces and scattered by an impact upon collision with the cementing component 12. Thus, the resin coat 25 is removed from a part of each particle 23 which is embedded into the cementing component 12. Therefore, the particle 23 is strongly fixed to the cementing component 12. On the other hand, on and above the cementing surface 13, the most part of the resin coat 25 coating the particle 23 remains without being removed. In other words, each particle 23 is coated with the resin coat 25 except a part embedded into the cementing surface. Thus, the cementing object 11 has a resin-coated surface.

The cementing object 11 produced as mentioned above is bonded to the mating cementing object 31 illustrated in Fig. 3 via the resin-based cement 41. The resin coat 25 remaining on the surface of the particle 23 on and above the cementing surface 13 forms a chemical bond with the resin-based cement 41. Therefore, a strong bonding strength is achieved.

The cementing component 12 in the dental field is not limited to the crown mentioned above. For example, the cementing component 12 may be a bridge, an inlay, an implant super structure, a cast plate, or an orthodontic bracket, which is produced by the use of a dental alloy.

According to the above-mentioned method described in conjunction with the crown as the cementing component 12, the cementing object 11 comprising any other cementing component 12 can be similarly produced and exhibits a strong bonding strength when it is bonded to the mating cementing object 31 via the resin-based cement 41.

Generally, the mating cementing object 31 is a tooth in case where the cementing component 12 to be bonded thereto is a bridge, an inlay, or an orthodontic bracket. The mating cementing object 31 is an implant in case where the cementing component 12 to be bonded thereto is an implant super structure. The mating cementing object 31 is a plate resin in case where the cementing component 12 to be bonded thereto is a cast plate.

As described above, a part of each particle 23 is embedded into the cementing surface 13 when the powder 27 is sprayed, so that the particle 23 is strongly fixed to the cementing component 12. Therefore, the material of the particles 23 is selected depending upon the material of the cementing component 12.

For example, the particles 23 may be alumina, glass beads, or silicon carbide (SiC), which is harder than the cementing component 12.

The cementing object 11 according to the embodiment of this invention exhibits a high bonding strength. In order to prove the high bonding strength achieved by this invention, an experimental test was performed using comparative samples.

In the comparative samples, powder as an aggregate of particles and resin powder were mixed at various mixing ratios. As each of a cementing component and a mating cementing object, a metal plate was prepared.

In the comparative samples, the powder comprises the particles and the resin powder but the particles were not coated with a resin coat.

Preparation was made of three kinds of powder, i.e., alumina, glass beads, and silicon carbide. Each powder was mixed with the resin powder to obtain a mixture. The metal plate as the cementing component was subjected to sandblasting by directly spraying the mixture onto a cementing surface of the metal plate. Thus, a cementing object comprising the metal plate with the mixture attached thereto was obtained. Thereafter, the cementing object or the cementing surface of the metal plate was bonded to another metal plate as the mating cementing object. Then, the bonding strength was measured.

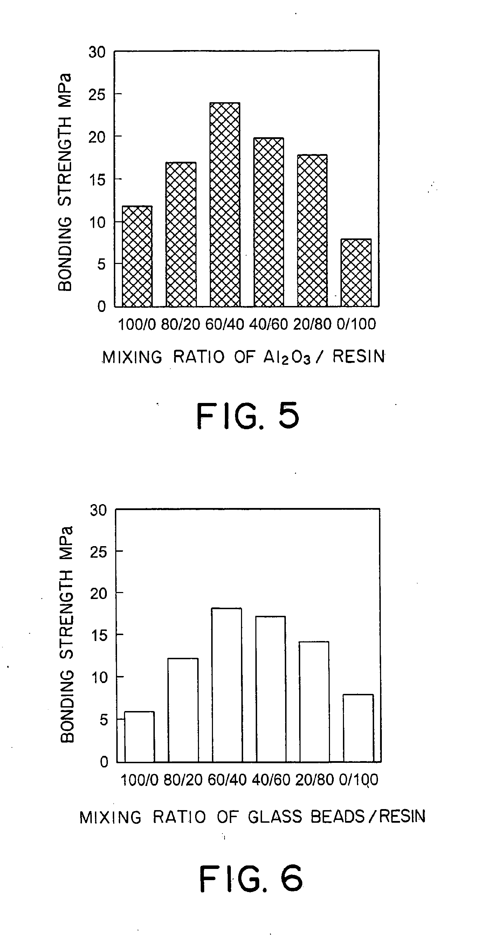

Referring to Fig. 5, the cementing object of a comparative sample 1 was obtained by spraying a mixture of alumina powder and resin powder onto the metal plate. After the cementing object was bonded to the mating cementing object via the resin-based cement, the bonding strength was measured.

As seen from Fig. 5, the bonding strength was maximum and equal to 24 MPa when the mixing ratio of the alumina powder and the resin powder was 60/40.

Referring to Fig. 6, the cementing object of a comparative sample 2 was obtained by spraying a mixture of glass beads and resin powder onto the metal plate. After the cementing object was bonded to the mating cementing object via the resin-based cement, the bonding strength was measured.

As seen from Fig. 6, the bonding strength was maximum and equal to 18 MPa when the mixing ratio of the glass beads and the resin powder was 60/40.

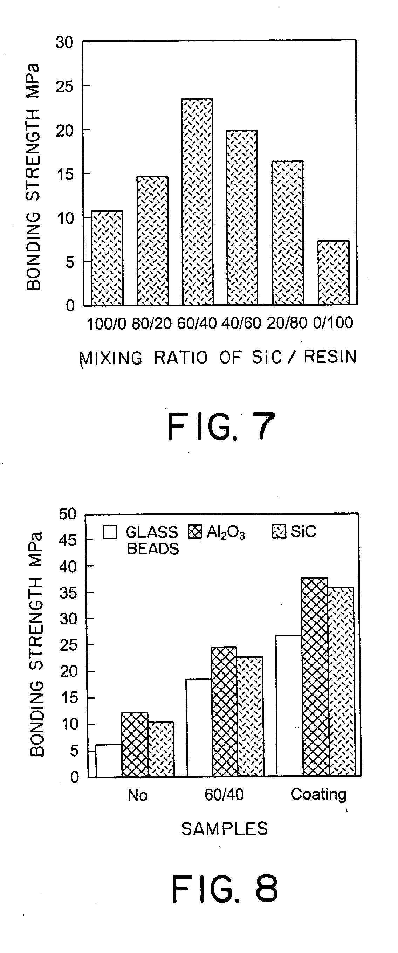

Referring to Fig. 7, the cementing object of a comparative sample 3 was obtained by spraying a mixture of SiC powder and resin powder onto the metal plate. After the cementing object was bonded to the mating cementing object via the resin-based cement, the bonding strength was measured.

As seen from Fig. 7, the bonding strength was maximum and equal to 23 MPa when the mixing ratio of the SiC powder and the resin powder was 60/40.

Thus, in each of the comparative samples 1 to 3, the maximum bonding strength was achieved at the mixing ratio of 60/40.

Referring to Fig. 8, comparison in bonding strength will be made between the cementing object according to this invention and the cementing objects of the comparative examples.

In addition to the comparative examples 1 to 3 mentioned above, a cementing object of a comparative sample 4 was prepared. The cementing object of the comparative sample 4 comprises a metal plate as a cementing component and resin-free powder attached thereto. Three kinds of resin-free powder, i.e., glass beads powder, alumina powder, and SiC powder were used. In Fig. 8, the comparative sample 4 is labeled "No". The comparative samples 1 to 3 at the mixing ratio of 60/40 are labeled "60/40". The cementing object according to this invention is labeled "Coating".

As the cementing object of this invention, three kinds of powder, i.e., glass beads powder, alumina powder, and SiC powder were prepared. Each of the powder and resin powder were mixed at a mixing ratio of 60/40. After the powder was coated with the resin coat to produce resin-coated powder, the resin-coated powder was sprayed onto a cementing surface of a metal plate as a cementing component to obtain the cementing object. The cementing object was bonded to another metal plate as a mating cementing object via a resin-based cement.

As seen from Fig. 8, the bonding strength of the cementing object of this invention was as follows.

Specifically, the glass beads powder coated with the resin coat had the bonding strength of 26 MPa. The alumina powder coated with the resin coat had the bonding strength of 37 MPa. The SiC powder coated with the resin coat had the bonding strength of 35 MPa.

Thus, it has been confirmed that the cementing object according to this invention had a considerably high bonding strength as compared with the cementing objects in the comparative samples 1 to 4.

As a result of the experimental test, it has been revealed that the mixing ratio of the powder and the resin powder is preferably equal to about 60/40 also in case where the particle is coated with the resin coat.

The cementing component comprising a metal or an alloy is not limited to a dental object. According to this invention, the bonding strength can be improved for any cementing component to be bonded to a mating cementing object via a resin-based cement.

For example, this invention is applicable to bonding between a cementing component and a mating cementing object each of which is made of a metal or an alloy, bonding between a cementing component made of a metal or an alloy and a ceramic material as a mating cementing object, and so on.

In the foregoing, the cementing object is coated with the resin coat. In addition, the mating cementing object may be coated with the resin coat before the cementing object and the mating cementing object are bonded via the resin-based cement.

While this invention has thus far been described with reference to the preferred embodiment thereof, it will be readily possible for those skilled in the art to put this invention into practice in various other manners without departing from the scope of this invention.

## Claims

1. A cementing object to be bonded to a mating cementing object via a resin-based cement, said cementing object comprising:
a cementing component which is formed by a metal or an alloy and which has a cementing surface;
powder particles fixedly attached to said cementing component with the powder particles partially embedded into the cementing surface of said cementing component; and
a resin coat coating a surface of each particle except a part embedded into the cementing surface.

2. The cementing object according to claim 1, wherein said powder particles are one of silicon carbide, glass beads, and alumina.

3. The cementing object according to claim 1 or 2, wherein said cementing object is a dental object and said cementing component is selected from a group including a crown or a bridge as a prosthetic restoration, an inlay for restoration, an implant super structure, a cast plate, and an orthodontic bracket each of which is made of a metal or an alloy.

4. A method of producing a cementing object to be cemented to a mating cementing object via a resin-based cement, said method comprising the steps of:
preparing a cementing component which is formed by a metal or an alloy and which has a cementing surface;
preparing resin-coated powder particles by mixing powder particles with resin; and
spraying said resin-coated powder particles onto the cementing surface of said cementing component to fixedly attach said resin-coated powder particles to said cementing component with said particles partially embedded into said cementing surface.

5. The method according to claim 4, wherein said powder particles are one of silicon carbide, glass beads, and alumina.

6. The method according to claim 4 or 5, wherein said cementing object is a dental object and said cementing component is selected from a group including a crown or a bridge as a prosthetic restoration, an inlay for restoration, an implant super structure, a cast plate, and an orthodontic bracket each of which is made of a metal or an alloy.

7. Cementing powder for use in a dental cementing object, preferably for use in a cementing object according to claim 1, 2 or 3, comprising powder particles of silicon carbide, glass beads, or alumina, and resin coats coating the powder particles.
